# EUROPÄISCHE PATENTANMELDUNG

(11) **EP 2 545 932 A1**
(43) Veröffentlichungstag der Anmeldung: **16.01.2013**
(21) Anmeldenummer: 11173710.2
(22) Anmeldetag: 12.07.2011
(51) Int. Cl.: A61K 36/71, A61P 19/00, A61P 19/10

(54) **Ausgewählte Cimicifuga-Fraktionen zur Behandlung von Osteoporose**

(71) Anmelder: BIONORICA SE, 92318 Neumarkt (DE)
(72) Erfinder: Intelmann, Daniel., 6020 Innsbruck (AT); Schuh, Markus., 90419 Nürnberg (DE); Stecher, Guenther., 6091 Götzens (AT); Abel, Gudrun., 91301 Forcheim (DE); Jakob, Franz., 97082 Würzburg (DE); Ebert, Regina., 97241 Bergtheim (DE); Wuttke, Wolfgang., 37120 Bovenden (DE); Seidlovà-Wuttke, Dana., 37120 Bovenden (DE)
(74) Vertreter: Simandi, Claus

(57) **Zusammenfassung**

Die Erfindung betrifft Cimicifuga-Fraktionen zur Prophylaxe und Behandlung von Osteoporose am Mensch und Tier, Arzneimittel davon und Verfahren zu deren Herstellung.

## Beschreibung

Die Erfindung betrifft Cimicifuga-Fraktionen zur Prophylaxe und Behandlung von Osteoporose am Mensch und Tier, Arzneimittel davon und Verfahren zu deren Herstellung.

Cimicifuga-Extrakte sind zur Behandlung von Krankheiten auf der Basis von wäßrig-ethanolischen Auszügen bereits bekannt. Insbesondere deren Trockenextrakte werden als Phytopharmaka eingesetzt.

In den vergangenen Jahren war der Einsatz der Hormonersatztherapie (HRT) bei klimakterischen postmenopausalen Beschwerden wie Gemütsschwankungen, Hitzewallungen und vermehrtes Schwitzen weit verbreitet. Die Zunahme kardiovaskulärer und thromboembolischer Erkrankungen sowie erhöhtes Brustkrebsrisiko während der Behandlung mit HRT indizieren die Suche nach einer Medikation ohne die estrogenabhängigen Nebenwirkungen. Die Anmelderin vertreibt daher das Cimicifuga-Arzneimittelprodukt Klimadynon^{®} (Trockenextrakt aus Cimicifugawurzelstock) zur Behandlung von klimakterischen Beschwerden.

Cimicifuga-Extrakte sind als östrogenartiges organselektives Arzneimittel bereits in WO1999047149A1 beschrieben. WO200236140A offenbart, dass Zubereitungen von Cimicifuga beim Vorliegen einer Harninkontinenz therapeutisch wirksam sind.

Weiterhin wird in WO1999047149A1 die Eignung von Cimicifuga-Extrakten als organselektives Arzneimittel zur Behandlung von Osteoporose und Artheriosklerose offenbart.

Es besteht jedoch ein hohes Interesse Cimicifuga Extrakte zu optimieren und verbesserte (Spezial)Extrakte bereitzustellen.

Daher besteht die erfindungsgemäße Aufgabe ein verbessertes Arzneimittel zur Behandlung von Osteoporose auf der Basis von Cimicifuga bereitzustellen.

Überraschender Weise konnte gefunden werden, dass bestimmte Cimicifuga-Fraktionen zur Behandlung und Prophylaxe von Osteoporose besonders geeignet sind.

Im WO1999047149A1 ist beschrieben, dass mit organischen Lösungsmitteln (Dichlormethan) mittels Liquid-Liquid-Extraktion gegen Wasser ein organo-selektiver Extrakt aus der organischen Phase erhalten werden kann, während die wässrige (Rest)Phase keine Aktivität zeigt (Nachweis durch einen Estradiol-Bindungsassay). Ein solcher organo-selektiver Extrakt ist für die Therapie bei Estrogenmangel geeignet, einschließlich zur Behandlung der Osteoporose.

Ausgehend von diesem Stand der Technik konnten überraschender Weise mittels einem chromatographischen Schritt Fraktionen erhalten werden, die eine knochenprotektive und anti-osteoporische Wirkung zeigen und zwar sowohl für die wässrige Restphase als auch für die organische Phase.

In einer bevorzugten Ausführungsform erfolgt der chromatographische Schritt anhand einer festen (stationären) Phase, insbesondere mittels einer Säulenchromatographie, bzw. Phasenchromatographie, insbesondere eine Umkehrphasenchromatograhie, insbesondere Normaldruck-Silica-Umkehrphasenchromatograhie wie z.B. mittels einer RP-C18 Säule. In einer bevorzugten Ausführungsform kann eine Hochleistungsflüssigkeitschromatographie (HPLC) durchgeführt werden.

Der chromatographische Schritt erlaubt die Bereitstellung von Fraktionen, die einen hohen Anteil an wirksamen Inhaltsstoffen aufweisen, die zudem vorteilhaft angereichert sind, so dass eine wirksame Dosis mit geringerem Gehalt gegenüber dem Stand der Technik erreicht werden kann. Zudem erlaubt der erfindungsgemäße chromatographische Schritt eine verbesserte Reproduzierbarkeit und Uniformität der erhaltenen erfindungsgemäßen Fraktionen.

Die Erfindung betrifft daher ein Verfahren zur Herstellung von knochenprotektiven und anti-osteoporischen Cimicifuga-Fraktionen aus einem Cimicifuga Extrakt, wobei mindestens ein chromatographischer Schritt durchgeführt wird.

Weiterhin ist bevorzugt, dass in einem ersten Schritt eine Liquid-Liquid-Extraktion (kurz: LLE) von Cimicifuga erfolgt, wobei ein erstes Lösungsmittel Wasser ist oder wässrige Anteile aufweist (wie z.B. eine Mischung aus Wasser und Alkohol, 70:30 v/v, 50:50 v/v, 30:70 v/v) und das zweite Lösungsmittel ein organisches Lösungsmittel ist. Organische Lösungsmittel können vorzugsweise solche sein, wie Dichlormethan, Ether, Diethlyether, tert. Butylmethyether (TBME), Pentan, Hexan, Heptan. Es werden erfindungsgemäß eine wässrige Fraktion ("C001/R") und eine lipophile Fraktion ("C001/S") erhalten.

Daher betrifft die Erfindung ein Verfahren zur Herstellung von knochenprotektiven und anti-osteoporotischen Cimicifuga-Fraktionen, wobei in einem ersten Schritt
i.) eine Liquid-Liquid-Extraktion von Cimicifuga erfolgt und
ii.) die erhaltenen Fraktionen mit mindestens einem chromatographischen Schritt aufgetrennt und auf diese Weise die knochenprotektiven und anti-osteoporotischen Cimicifuga-Fraktionen erhalten werden.

Weiterhin betrifft die Erfindung ein Verfahren zur Herstellung von knochenprotektiven und anti-osteoporotischen Cimicifuga-Fraktionen, wobei in einem ersten Schritt
i.) eine Liquid-Liquid-Extraktion von Cimicifuga mit einer wässrigen Phase gegen eine organische Phase erfolgt und
ii.) die erhaltenen Fraktionen mit mindestens einem chromatographischen Schritt aufgetrennt und auf diese Weise die knochenprotektiven und anti-osteoporotischen Cimicifuga-Fraktionen erhalten werden.

In einer weiteren Ausführungsform kann der chromatographische Schritt wiederholt werden.

Ferner ist bevorzugt, dass die wässrige Phase hohe Anteile an Wasser sowie einen pH Wert von 7 bis 9 aufweisen, vorzugsweise 8 bis 8,5. Sofern erforderlich kann der pH Wert mittels einer Base eingestellt werden.

In einer weiteren Ausführungsform der Erfindung kann der chromatographischen Schritt vorzugsweise an der jeweiligen wässrigen oder lipophilen Fraktion erfolgen.

Besonders vorteilhaft können solche (Sub)Fraktionen aus der lipophilen Fraktion erhalten werden, die Saponine, Actein und / oder Deoxyactein enthalten oder mit Saponinen, Actein und / oder Deoxyactein angereichert sind.

Besonders vorteilhaft können solche (Sub)Fraktionen aus der wässrigen Fraktion erhalten werden, die Cimipronidin oder Cyclocimipronidin enthalten oder mit Cimipronidin oder Cyclocimipronidin angereichert sind.

Ferner sind sämtliche (Sub)Fraktionen bevorzugt, die Cimipronidin oder Cyclocimipronidin enthalten und mit Cimipronidin oder Cyclocimipronidin angereichert sind, auch wenn Saponine, Actein und / oder Deoxyactein vorliegen.

Überraschender Weise zeigen Fraktionen, die mit Saponinen, Actein und / oder Deoxyactein, Cimipronidin, Cyclocimipronidin angereichert sind, eine besonders vorteilhafte knochenprotektive und anti-osteoporotische Wirkung.

Dies ist besonders überraschend für die Fraktionen aus der wässrigen Phase einer Liquid-Liquid Extraktion, da im Stand der Technik die wässrige Fraktion keine Aktivität aufweist, jedoch die erfindungsgemäßen (Sub)Fraktionen aus der wäßrigen Phase eine knochenprotektive und anti-osteoporotische Wirkung zeigen können.

Darüber hinaus zeigen die erfindungsgemäßen (Sub)Fraktionen aus der wäßrigen Phase eine vorteilhafte Wirkung gegen Hitzewallungen ("Hot Flushes"), wie z.B. für peri- oder postmenopausalen Beschwerden bzw. klimakterischen Beschwerden beschrieben.

Daher betrifft die Erfindung ebenfalls ein Arzneimittel enthaltend Cimicifuga-Fraktionen zur Behandlung und Prophylaxe von Osteoporose am Mensch und Tier, wobei mindestens ein chromatographischer Schritt durchgeführt wird.

Im Rahmen dieser Erfindung wird unter dem Begriff "Cimicifuga" (Traubensilberkerze) eine aus ursprünglich Nordamerika stammende (Arznei)Pflanze *Cimicifuga* racemosa (CR) - auch indianische Frauenwurzel genannt - verstanden, diese ist ca. 2 m groß und besitzt silbrig-weiße Blüten die lange Trauben bilden. Arzneilich verwendet wird der getrocknete Wurzelstock mit den Wurzeln (Droge). Erfindungsgemäß umfasst sind ebenfalls die folgenden Arten: *C*. *foetida*, *C*. *dahurica*, *C*. *heracleifolia*, *C*. *simplex, C*. *japonica, C*. *europaea, C*. *acerina, C*. *elata, C*. *rubifolia.* Weiterhin können Rhizome, Wurzeln, Stengel, Blätter und/oder Blütenblätter der Pflanzen verwendet werden.

Der Fachmann ist in der Lage aus Cimicifuga Pflanzenteile, insbesondere Wurzel, geeignete "Cimicifuga Extrakte" nach Anspruch 1 herzustellen, wie bereits kommerziell erhältlich, z.B. Klimadynon^{®} (supra). In der Literatur ist ein geeigneter (wässrig-ethanolischer) Extrakt als "CR BNO 1055" beschrieben. Z.B. können erfindungsgemäße Auszüge oder Fraktionen mittels organischen Lösungsmitteln, wie Alkohole, insbesondere Ethanol, Dichlormethan, Heptan u.a. erhalten werden. Weiterhin sind wässrige Auszüge oder Fraktionen möglich. Ebenfalls erfindungsgemäß umfasst sind entsprechende Trockenextrakte, wie z.B. das bekannte "BNO 1055".

Beschriebene Inhaltsstoffe von Cimicifuga bzw. Cimicifuga-Extrakten können sein: Triterpene, wie Acetol- und Cimicigenolderivate, Polyphenole, wie Kaffeesäure und deren Derivate, Chlorogensäure, Piscidinsäure, Fucinolsäure, Ferula-und Isoferulasäure, Cimicifuginsäuren, Formononetin, Alkaloide u.a..

Die Cimicifuga-Fraktionen können durch das erfindungsgemäße Verfahren hergestellt werden.

Daher betrifft die Erfindung Cimicifuga-Fraktionen erhältlich durch ein erfindungsgemäßes Verfahren oder die Cimicifuga-Fraktionen werden durch ein erfindungsgemäßes Verfahren erhalten.

Daher betrifft die Erfindung ein Verfahren zur Herstellung eines Arzneimittels (Medikament) bzw. pharmazeutische Zusammensetzung oder die Verwendung eines Arzneimittels (Medikament) bzw. pharmazeutische Zusammensetzung enthaltend Cimicifuga-Fraktionen zur Behandlung und Prophylaxe von Osteoporose am Mensch und Tier, ggfs. weitere Hilfs- und Zusatzstoffe.

Im Rahmen dieser Erfindung wird unter "Osteoporose" die Erkrankung des Skelettsystems mit Verlust bzw. Verminderung von Knochensubstanz und -struktur und erhöhter Frakturanfälligkeit verstanden. Zur weiteren Definition siehe z.B. Pschyrembel, de Gruyter, 261. Auflage, Berlin 2007.

Daher betrifft die Erfindung ebenfalls ein erfindungsgemäßes Arzneimittel zur Behandlung und Prophylaxe von Osteoporose enthaltend Cimicifuga-Fraktionen zur Behandlung und Prophylaxe von Osteoporose am Mensch und Tier, ggfs. weitere Hilfs- und Zusatzstoffe.

Ferner betrifft die Erfindung daher eine pharmazeutische Formulierung enthaltend ein erfindungsgemäßes Arzneimittel (auch "Wirkstoff" oder "Zusammensetzung" genannt).

Beispiele für besonders geeignete pharmakologisch verträgliche Träger sind dem Fachmann bekannt und umfassen gepufferte Kochsalzlösungen, Wasser, Emulsionen wie z.B. ÖI/Wasser-Emulsionen, verschiedene Arten von Detergentien, sterile Lösungen, etc.

Pharmazeutische Zusammensetzungen, die solche Träger umfassen, können mittels bekannter konventioneller Methoden formuliert werden. Diese pharmazeutischen Zusammensetzungen können einem Individuum / Patienten in einer geeigneten Dosis verabreicht werden. Die Verabreichung kann oral, parenteral, intravenös, intraperitoneal, subcutan, intramuskulär, lokal, intranasal, oder intradermal, oder über einen Katheter an einer Stelle in einer Arterie erfolgen. Die Art der Dosierung wird vom behandelnden Arzt entsprechend den klinischen Faktoren bestimmt.

Im Rahmen dieser Erfindung ist die orale und subcutane Applikation bevorzugt.

Es ist dem Fachmann bekannt, dass die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden.

Die erfindungsgemäßen Arzneimittel können in Form von pharmazeutischen Zubereitungen in Dosierungseinheiten zubereitet werden. Dies bedeutet, dass die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben, einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Formulierungen seien Tabletten, Dragees, Kapseln, Pillen, Granulate, Suppositorien, Lösungen, Saft, Suspensionen und Emulsionen, Pasten, Salben, Gele, Cremes, Lotions, Puder und (Nasen)Sprays genannt. Tabletten, Dragees, Kapseln, Pillen und Granulate können den oder die Wirkstoffe neben den üblichen Trägerstoffen enthalten, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Weiterhin ist bevorzugt, dass die galenische Form eine Manteltablette, insbesondere eine Calcium-Manteltablette ist, wie in WO2002100422 offenbart.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. C14-Alkohol mit C16-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant- Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3- Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten. Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Zur Herstellung der erfindungsgemäßen Cimicifuga - Extrakte und wird zudem auf die technischen Lehren, die Gegenstand von EP 1368605B1, EP 0753306B1 sind, verwiesen.

Nachfolgende Beispiele und Figuren dienen zur Erläuterung der Erfindung, ohne die Erfindung auf diese Beispiele zu beschränken.

Beispiele:
Der Cimicifuga racemosa Trockenextrakt (C001) wurde über Dichlormethan-Wasser (pH 8,5) Verteilung in zwei Fraktionen aufgearbeitet. Die Wasserfraktion (C001/R) beinhaltet sowohl die einfachen Phenylpropanoide als auch die Cimicifugasäuren, während in der Dichlormethanphase (C001/S) die Saponine angereichert sind.

Ausgehend vom Trockenextrakt finden sich 65 % der Inhaltsstoffe in der wasserlöslichen Phase und 20 % der Inhaltsstoffe in der lipophilen Phase wieder. Die auf 100 % fehlenden 15 % der Inhaltsstoffe finden sich in der Mischphase (entspricht der Grenzschicht zwischen den beiden nicht mischbaren Flüssigkeiten während der flüssig-flüssig Aufarbeitung), welche analytisch untersucht aber nicht weiter aufgearbeitet wurde. Diese 15 % entsprechen in ihrer Zusammensetzung dem Gesamtextrakt.

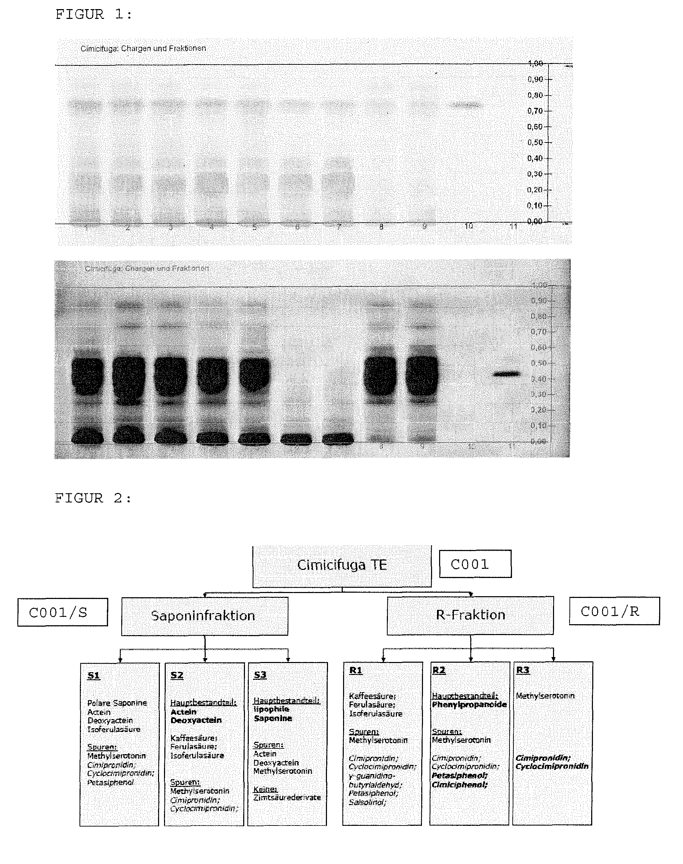

Die dünnschichtchromatographische Untersuchung der hergestellten Fraktionen ist in FIGUR 1 dargestellt. In der wasserlöslichen Fraktion findet man sowohl einfache (Rf bei 0,7-0,8) als auch zusammengesetzte Phenylpropanoide (Rf bei 0,2-0,3) vor (Kontrolle mit Dünnschichtchromatographie). Weiterhin konnte über massenspektroskopische Analyse auch die Anwesenheit von Methylserotonin bestätigt werden.

In der lipophilen Fraktion findet man Saponine wieder. Dies wurde sowohl über Dünnschichtchromatographie als auch über HPLC-MS bestätigt.

FIGUR 1 zeigt ebenfalls einen analytischen Vergleich zwischen verschiedenen, zu unterschiedlichen Zeitpunkten hergestellten Cimicifuga Extrakten (Bahn 1 - 5).

Die in FIGUR 1 dargestellten Fraktionen (LLE-Fraktion) und zwar wasserlösliche Fraktion (= Restfraktion; C001/R) und lipophile Fraktion (=Saponinfraktion; C001/S) wurden einer weiteren Subfraktionierung mittels einem chromatographischen Schritt unterzogen und die Subfraktionen wurden auf deren knochenprotektive und anti-osteoporische Wirkung hin getestet. Die Subfraktionierung erfolgt mittels einer Aufarbeitung der LLE-Fraktionen über Normaldruck-Silica-Umkehrphasenchromatographie. Die gewonnenen Subfraktionen wurden analytisch geprüft und schließlich jeweils in drei charakteristische Pools vereint (C001/R1, C001/R2, C001/R3 und C001/S1, C001/S2, C001/S3).

### Gewinnung von Subfraktionen aus Saponin- und Restfraktion mittels Säulenchromatographie

Die beiden Fraktionen (C001S, C001R) werden zu jeweils 3 Subfraktionen weiterverarbeitet. Hierzu werden die Fraktionen auf eine mit Silicagel RP-C18 gepackte Glassäule aufgegeben und anschließend mit steigender Methanolkonzentration eluiert. Über einen Fraktionskollektor werden je 20 ml Aliquots gesammelt, die per Dünnschichtchromatographie untersucht wurden. Basierend auf den Ergebnissen der Dünnschichtchromatographie wurden die Aliquots gepoolt. Nach Einengen der Pools am Rotavapor werden diese gefroren und am Lyophilisator getrocknet, wodurch die Subfraktionen gewonnen wurden. Das Fraktionierungsprotokoll sieht im Detail wie folgt aus:
Die Saponinfraktion wird in Methanol (1 ml pro Gramm Saponinfraktion) angelöst, dann die gleiche Menge Wasser zugegeben und die entstehende Suspension auf die Säule aufgegeben. Eluiert wird nacheinander mit 50 %, 60 %, 80 % und 100% Methanol. Man erhält die 3 Subfraktionen S1 (26 % der eingesetzten Menge an Saponinfraktion), S2 (37 %), und S3 (37 %).

Die Restfraktion wird in 10 % Methanol suspendiert (1,25 ml pro Gramm Restfraktion) und in dieser Form auf die Säule aufgegeben. Eluiert wird nacheinander mit 10 %, 30 % und 70 % Methanol. Nach Vereinen der entsprechenden Aliquots liegen die 3 Subfraktionen R1 (72 % der eingesetzten Restfraktion), R2 (19 %), und R3 (9 %) vor.

Entsprechende Kontrollen wurden mit Dünnschichtchromatographie durchgeführt.

**Tabelle 1 Inhaltsstoffe in den Cimicifuga Fraktionen**

| **MW** | **Substanz** | **gefunden** | **C001/S1** | **C001/S2** | **C001/S3** | **C001/R1** | **C001/R2** | **C001/R3** |
|---|---|---|---|---|---|---|---|---|
| 129 | Gamma-Guanidinobutyraldehyd | + | | | | ++ | | |
| 153 | Cyclocimipronidin | + | + | ++ | | ++ | | + |
| 171 | Cimipronidin | + | + | + | | ++ | ++ | +++ |
| 180 | Kaffeesaure | + | | | | | | |
| 182 | Monohydroxyterephtalsaure | | | | | | | |
| 183 | Salsolinol | | | | | + | | |
| 185 | Methylcimipronidin | | | | | | | |
| 190 | N **ω** -methylserotonin | + | | | | | | +++ |
| 194 | Isoferulasaure | + | | | | | | |
| 194 | Ferulasäure | + | | | | | | |
| 207 | "falsches" N **ω** -methylserotonin | | | | | | | |
| 256 | Piscidinsaure | | | | | | | |
| 272 | Fukiinsaure | | | | | | | |
| 284 | Kaffeesaurephenetyhlester | | | | | | | |
| 291 | Dopargin | | | | | | | |
| 330 | Petasiphenon | | | | | | | |
| 344 | Petasiphenol | + | + | | | + | +++ | |
| 344 | Cimiphenon | + | + | | | | | |
| 358 | Cimiphenol | + | | | | | +++ | |
| 400 | Alpha-Peltatin | - | | | | | | |
| 400 | Podophyllotoxin | - | | | | | | |
| 414 | Beta-Peltatin | - | | | | | | |
| 418 | Cimicifugasaure D | + | | | | | +++ | |
| 432 | Cimicifugasaure E | | | | | | | |
| 432 | Cimicifugasaure F | | | | | | | |
| 434 | Fukinolsaure | + | | | | | +++ | |
| 447 | Cimicifugasaure A | + | | | | | ++ | |
| 447 | Cimicifugasaure B | + | | | | | | |

STRUTS Analyse der Cimicifuga Fraktionen (insb. C001/R1, C001/R2 und C001/R3 und C001/S1, C001/S2 und C001/S3):
Mit der Struts-Analyse wird die Konnektivität des trabekulären Knochens bestimmt. Als Maß hierfür dient das Verhältnis zwischen den Trabekel-verknüpfungspunkten (NODES) und den Trabekelenden (STRUTS). Je mehr Nodi und je weniger freie Trabekelenden bestimmt werden können, desto stabiler ist das Bälkchenknochen-Netzwerk.

Diese Methode ist hilfreich, um über die Konnektivität der Spongiosa eine gezieltere Aussage treffen zu können. Die trabekuläre Struktur wird zu Knotenpunkten (NODES) und Strichen (STRUTS) vereinfacht. Ein Knoten ist definiert als ein Punkt der Verknüpfung von drei oder mehreren Trabekeln. Ein freies Ende ist definiert als ein Punkt an dem kein Zusammentreffen von Trabekeln stattfindet. Struts, als die individuellen Trabekel, werden als Prozent der totalen Strut-Länge ausgedrückt.

### Parameter der Node-Strut-Analyse:

- Anzahl der Trabekelknotenpunkte (No.Nodes [#/])
- Anzahl der freien Trabekelenden [#/mm2]
- Totale Strut-Länge (TSL [mm/ mm2])
- Strut-Länge von freien Trabekelenden zu freien Trabekelenden (SL.FF [% of TSL])
- Strut-Länge von Trabekelknotenpunkten zu Trabekelknotenpunkten (SL.NN [% of TSL])

### Design:

■ 10 Versuchstiergruppen ä 10 Tiere
■ orale Gabe von:
   a) den jeweiligen Gewichtsäquivalenten der Fraktionen/Unterfraktionen (C001/S, C001/S/1-3, C001/R und C001/R1-3) in Bezug auf C001 Gesamtextrakt (10 mg Gesamtextrakt/Tier)
   b) Negativ-Kontrolle: phytoestrogenarmes Futter ("Ko")
   c) Positiv-Kontrolle: Estradiolbenzoat 10 mg/kg Tier ("E2")

Behandlungsdauer: 4 Wochen

Haupt-Parameter: Gewichtszunahme, Futteraufnahme, Knochendichte

### Ergebnisse:

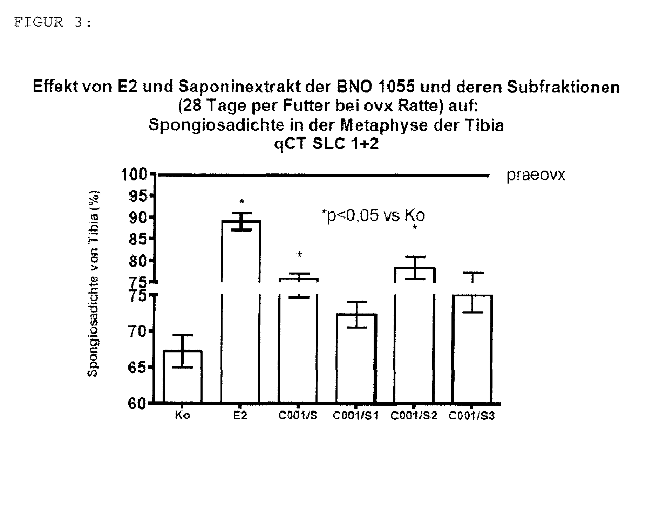

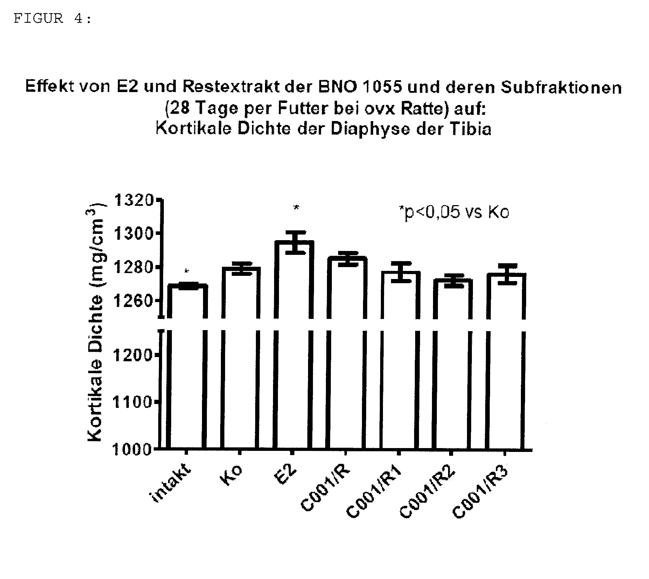

a.) Es konnte gezeigt werden, dass die Saponinfraktion (C001/S) knochenprotektiv (FIGUR 3) und die Restfraktion (C001/R) nicht knochenprotektiv wirkt (FIGUR 4),
b.) Die Fraktion C001/S/2 besitzt die stärkste knochenprotektive Aktivität der drei Saponinsubfraktionen (FIGUR 3),
c.) Die Dritte von den 3 Subfraktionen der Restfraktion (C001/R/3) zeigt eine starke knochenprotektive Aktivität (FIGUR 4).

Mit einem Quantitativen Computertomograph (qCT) ist eine schnelle und genaue Ermittlung der Knochenmineraldichte von Ratten zu erreichen. Der zuerst in der Spongiosa der Extremitätenknochen ovarektomierter Ratten einsetzende Knochenverlust ist mit einem qCT schon bereits vier Wochen nach Ovarektomie deutlich festzustellen.

Im Ergebnis kann die potentielle knochenprotektive und anti-osteoporische Wirkung der Cimicifuga-Extrakte gezeigt werden.

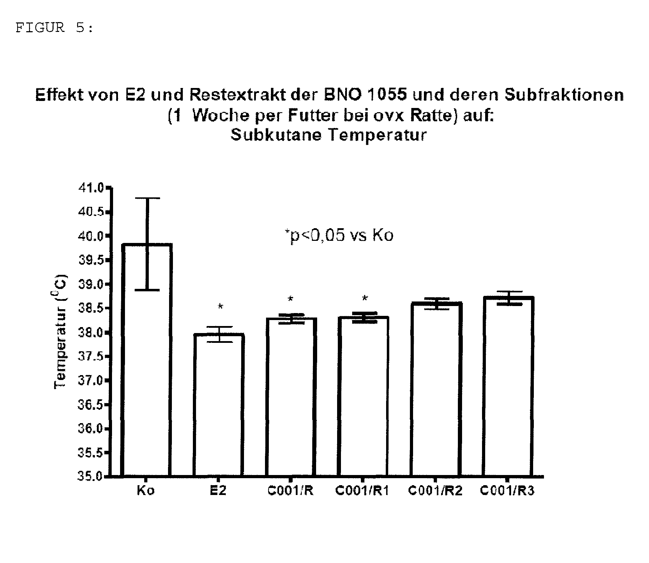

Durch Bestimmung der subkutanen Temperatur während des oben beschriebenen Rattenversuches nach Behandlung mit C001/S, C001/S/1-3 bzw. C001/R und C001/R/1-3 kann bestätigt werden, dass die Restfraktion und darin vor allem die Subfraktion C001/R und C001/R1-3 und nicht die Saponinfraktion samt Subfraktionen vorteilhaft die "hot-flushes" reduzieren können (FIGUR 5).

### Erläuterungen zu den Figuren:

Figur 1: Dünnschichtchromatographie unterschiedlicher Cimicifuga racemosa Trockenxtraktchargen und hergestellter Fraktionen. 1 = C002, 2 = C004, 3 = C003, 4 = C001, 5 = Charge 97042148, 6,7 = Wasserfraktion C001, 8,9 = lipophile Fraktion C002. 10 = Referenzstandard Isoferulasäure (Phenylpropanoid), 11 = Referenzstandard Deoxyactein (Saponin). Oben: UV-Detektion, Farben invertiert; Rf 0,2 - 0,3 Cimicifugasäuren, Rf 0,7-0,8 einfache Phenylpropanoide. Unten: Detektion nach Reaktion mit Anisaldhyd/Schwefelsäure. Rot gefärbte Banden entsprechen Saponinen.
Figur 2 gibt eine Übersicht zur durchgeführten Fraktionierung und zu den Hauptkomponenten, welche in den einzelnen Subfraktionen detektiert wurden. Legende: "Saponinfraktion (=C001/S)" ist die Dichlormethanfraktion (supra) und "R-Fraktion" ist die wässrige Fraktion (C001/R), "TE" = Trockenextrakt (Ausgangsextrakt = C001). Die (Sub-)Fraktionen C001/S1, C001/S2 und C001/S3 werden mittels einem chromatographischen Schritt aus C001/S erhalten (supra). Die (Sub-)Fraktionen C001/R1, C001/R2 und C001/R3 werden mittels einem chromatographischen Schritt aus C001/R erhalten (supra).
Figur 3 zeigt die mittels qCT gemessene Spongosiadichte in der Metaphyse der Tibia zu den Fraktionen und Subfraktionen gem. Legende nach Figur 2.
Figur 4 zeigt die mittels qCT gemessene Spongosiadichte in der Metaphyse der Tibia zu den Fraktionen und Subfraktionen gem. Legende nach Figur 2.
Figur 5 zeigt den Effekt der subkutanen Temperatur auf die ovx-Ratten zu den Fraktionen und Subfraktionen gem. Legende nach Figur 2.

## Patentansprüche

1. Verfahren zur Herstellung von knochenprotektiven und anti-osteoporischen Cimicifuga-Fraktionen aus einem Cimicifuga Extrakt, wobei mindestens ein chromatographischer Schritt durchgeführt wird.

2. Verfahren zur Herstellung von knochenprotektiven und anti-osteoporotischen Cimicifuga-Fraktionen nach Anspruch 1, wobei in einem ersten Schritt i.) eine Liquid-Liquid-Extraktion von Cimicifuga erfolgt und ii.) die erhaltenen Fraktionen mit mindestens einem chromatographischen Schritt aufgetrennt und die Fraktionen erhalten werden.

3. Verfahren zur Herstellung von knochenprotektiven und anti-osteoporotischen Cimicifuga-Fraktionen nach Anspruch 2, wobei in einem ersten Schritt i.) eine Liquid-Liquid-Extraktion von Cimicifuga mit einer wässrigen Phase gegen eine organische Phase erfolgt.

4. Verfahren zur Herstellung von knochenprotektiven und anti-osteoporotischen Cimicifuga-Fraktionen nach Anspruch 3, wobei die wässrige Phase hohe Anteile an Wasser aufweist und / oder die organische Phase aus der Gruppe Dichlormethan, Ether, Diethlyether, tert. Butylmethyether (TBME), Pentan, Hexan oder Heptan ausgewählt ist.

5. Verfahren zur Herstellung von knochenprotektiven und anti-osteoporotischen Cimicifuga-Fraktionen nach Anspruch 3, wobei die wässrige Phase einen pH Wert von 7 bis 9 aufweist, vorzugsweise 8 bis 8,5.

6. Verfahren zur Herstellung von knochenprotektiven und anti-osteoporotischen Cimicifuga-Fraktionen nach einem der vorhergehenden Ansprüche, wobei der chromatographische Schritt mittels einer festen Phase, insbesondere mittels Säulenchromatographie, Phasenchromatographie, Umkehrphasenchromatograhie, Normaldruck-Silica- Umkehrphasenchromatograhie oder Hochleistungsflüssigkeitschromatographie (HPLC) durchgeführt wird.

7. Cimicifuga Fraktion erhältlich nach einem der vorstehenden Ansprüche, ggfs. getrocknet und zerkleinert.

8. Cimicifuga Fraktion erhältlich nach einem der vorstehenden Ansprüche, **dadurch gekennzeichnet, dass** Saponine, Actein und / oder Deoxyactein enthalten oder angereichert sind und / oder Cimipronidin oder Cyclocimipronidin enthalten oder angereichert sind.

9. Pharmazeutische Zusammensetzung enthaltend eine Cimicifuga Fraktion nach einem der vorhergehenden Ansprüche, in Form von Pulver, Granulat, Tablette, Dragee, insbesondere eine Manteltablette oder Calcium-Manteltablette oder in einer galenischen Formulierung, insbesondere eine orale Formulierung wie Dragees, Tabletten, Filmtabletten, Kapseln, flüssige Verdünnungen, insbesondere Tropfen, Säfte, Sirupe oder eine Formulierung zur topischen Anwendung wie Sprays, Salben, Emulsionen, Puder, Pulver, flüssige oder feste Präparate für die Inhalation, Kompressen, oder Lyophilisat samt geeigneter Zusatz- und Hilfsstoffe.

10. Arzneimittel enthaltend eine Cimicifuga Fraktion nach einem der vorhergehenden Ansprüche zur Behandlung und Prophylaxe von Osteoporose am Mensch und Tier.
